Europäisches Patentamt

European Patent Office

Office européen des brevets

(11) Publication number: **0 162 813 B1**

# EUROPEAN PATENT SPECIFICATION

(45) Date of publication of patent specification: **11.03.92**   (51) Int. Cl.5: **C07K 5/06, A61K 37/02**

(21) Application number: **85810246.0**

(22) Date of filing: **24.05.85**

(54) Pharmaceutical compositions containing a dipeptide.

(30) Priority: **24.05.84 JP 105098/84**

(43) Date of publication of application:
**27.11.85 Bulletin 85/48**

(45) Publication of the grant of the patent:
**11.03.92 Bulletin 92/11**

(84) Designated Contracting States:
**AT BE CH DE FR GB IT LI LU NL SE**

(56) References cited:
**US-A- 3 976 773**

**AGENTS and ACTIONS, vol. 9, no. 4, 1979, pages 314-318, Birkhäuser Verlag, Basel, CH, L HAMMAR et al. : "Peptide inhibition of mammalian histidine decarboxylase"**

**AGENTS and ACTIONS, vol. 12, no. 112, 1982, pages 176-178 Birkhäuser Verlag, Basel, CH, M MATTSON etal. : "Effect of His-Phe, a competitive inhibitor of histidine decarboxylase on gastric acid secretion in chronic fistula rats : delay in acidsecretion response to pentagastrin"**

**R Berkow : The Merck Manual of Diagnosis and Therapy, 1982, publ. Merck Sharp &**

**Dohme Research Laboratories Chapter 52 "Peptic ulcer"**

**SCAND J GASTROENT. , 1974, vol. 9 pages 239-247. C E ELWIN : "Gastric acid responses to antral application of some amino acids, peptides, and isolated fractions of a protein hydrolysate**

(73) Proprietor: **NIPPON ZOKI PHARMACEUTICAL CO. LTD.**
**10, 2-chome Hirano-machi Higashi-ku Osaka-shi Osaka-fu(JP)**

(72) Inventor: **Konishi, Jin-emon**
**3-21-13, Higashi-machi Kichijohji Musashino-shi Tokyo(JP)**

(74) Representative: **Kerr, Andrew**
**Postfach 122 Finkelerweg 44 CH-4144 Arlesheim BL(CH)**

## Description

The present invention relates to pharmaceutical compositions containing a dipeptide or a pharmaceutically acceptable salt thereof and also to the use of said compositions, in particular for the treatment of ulcers.

Various peptides and derivatives thereof have been synthesized, and tested for pharmacological activity. After careful investigation of the pharmacological activities of various peptides it has now been found that certain dipeptides have useful pharmacological activity and in particular useful antiulcer activity.

The dipeptides of the present invention are in general known, but there is no prior mention of their possessing any pharmacological activity.

It is an object of the present invention to provide pharmaceutical compositions and in particular antiulcer compositions containing at least one dipeptide or a pharmaceutically acceptable salt thereof, as an active ingredient.

Peptic ulcers are conventionally treated by control of diet and the administration of antacids or histamine $H_2$ receptor blocking agents such as cimetidine (The Merck Manual of Diagnosis and Therapy, 14th. Edition, 727-780). Hammar et at., Agents and Actions, vol. 9, no 4, 1979,314-318 describe in vitro experiments showing activity of certain linear dipeptides having His at the amino terminus in suppressing the formation of histidine decarboxylase, which is stated to be involved in the biosynthesis of histamine. However it is stated (page 316, col. 1, lines 14, 15) that the non-hystidyl peptides tested were not inhibitory. Mattson et at., Agents and Actions, vol. 12, no. 112, 1982, 176-178 describe in vivo experiments showing that His-Phe is active in inhibiting histidine decarboxylase in chronic gastric fistula rats. However no observations of any effect on the growth of ulcers are reported.

According to the present invention there is provided a pharmaceutical composition, in particular an antiulcer composition containing as an active ingredient a dipeptide composed of two amino acid residues selected from glycine (Gly), serine (Ser), histidine (His) and lysine (Lys), or a pharmaceutically acceptable salt thereof, with the proviso that linear dipeptides having His at the amino terminus, and Gly-Gly are excluded.

The invention includes the use of a dipeptide composed of two amino acid residues selected from Gly, Ser, His and Lys, or a pharmaceutically acceptable salt thereof, with the proviso that linear dipeptides having His at the amino terminus, and Gly-Gly are excluded for the manufacture of pharmaceutical compositions, especially anti-ulcer agents.

Preferably the amino acid residue of the dipeptide at the amino (N-) terminus is Gly, or Ser and that at the carboxyl terminus is Ser, His or Lys, e.g. Gly-Ser, Ser-His. The dipeptide may also be a cyclic dipeptide, preferably cyclic His-Lys:

|-His-Lys-|

The amino acid residues in the invention can be any combination of the D-isomer and L-isomer.

The dipeptide used in the composition of the present invention can be synthesized by any convenient method, e.g. in the solid phase, the liquid phase or enzymatically. Furthermore, the dipeptide can also be obtained from natural products by extraction.

The pharmaceutical compositions of the present invention may contain the dipeptides as defined above as such, or in the form of pharmaceutically acceptable salts thereof. Examples of pharmaceutically acceptable salts which may be employed, include salts with an inorganic acid such as hydrochloric acid, sulfuric acid, nitric acid, hydrobromic acid, phosphoric acid, perchloric acid, thiocyanic acid or boric acid; salts with organic acids such as formic acid, acetic acid, a haloacetic acid, propionic acid, glycolic acid, citric acid, tartaric acid, succinic acid, gluconic acid, lactic acid, malonic acid, fumaric acid, anthranilic acid, benzoic acid, cinnamic acid, oxalic acid, maleic acid, p-toluenesulfonic acid, naphthalenesulfonic acid or sulfanilic acid; as well as salts with an alkali metal such as lithium, sodium or potassium, or an alkaline-earth metal such as calcium or magnesium as well as with any other suitable metals, such as aluminium.

The term "dipeptide" is defined herein as including metal complexes thereof, for example metal complexes with zinc, nickel, cobalt, copper, iron etc. The salts and metal complexes of the dipeptides may be prepared from the free dipeptides and/or converted to another salt or metal complex by any convenient method.

The pharmacological properties of the pharmaceutical compositions of the present invention will now be described in more detail below with reference to the following tests and examples.

1. Acute toxicity

2

A dipeptide as defined above was intravenously administered to mice and the acute toxicity was evaluated by observing mortality over a 72-hour period.

The $LD_{50}$ of the dipeptides employed in the compositions of the present invention was found to be in all cases higher than 2,000 mg/kg.

2. Inhibition of histamine-induced ulcer formation

A composition according to the present invention was intravenously administered (i.v.) to groups of 10 Wistar strain rats weighing 180 to 210 g which had been starved for 24 hours. Immediately thereafter, 300 mg/kg of histamine dihydrochloride was administered intraperitoneally (i.p.) F. Bürcher et al; [Beith. Path. Anat., 81, 391 (1928)]. Four hours later, each animal was bled to death by decapitation and the stomach removed. 7.5 ml of physiological saline was infused into the removed stomach and partially fixed in 10% formalin for 10 minutes. The stomach was then incised along its greater curvature. The sum of surface area of the ulcer lesions on the glandular part of the stomach was designated as the ulcer coefficient.

The results are given in Table 1.

## Table 1

| Composition of invention | Dosage (mg/kg) | Ulcer coefficient[d] | Inhibition rate (%) |
|---|---|---|---|
| None | 2.5ml/kg[a] | 45.0 ± 12.1 | - |
| Gly-Ser | 20 | 6.0 ± 4.3 | 86.6 |
| Gly-Ser | 50[c] | 18.6 ± 7.3 | 58.6 |
| Ser-His | 20 | 17.1 ± 8.5 | 62.1 |
| [His-lys][b] | 20 | 14.9 ± 6.0 | 66.8 |
| Cimetidine * | 20 | 19.1 ± 10.6 | 57.6 |

(a) physiological saline          (b) cyclic histidyllysine

(c) oral administration          (d) mean ± S.E.     * Control

As may be seen from Table 1, the dipeptide active ingredients of the present invention possess a significant antiulcer activity. Thus, the pharmaceutical compositions of the present invention are useful in the preventive medication of, or for the treatment of, ulcers of peptic organs, such as stomach and duodenum, aphthous stomatitis, scalds, burns, etc.

The pharmaceutical compositions of the present invention have the advantages that the dipeptides or salts or complexes thereof have low toxicity and give low incidence of side effects.

The pharmaceutical compositions of the present invention can be formulated in any convenient manner using an appropriate carrier or diluent. The compositions may be in solid, semisolid or liquid form and be presented in a conventional manner for oral or parenteral administration.

In pharmaceutical dosage form the compositions of the invention may contain the dipeptide per se or in the form of a pharmaceutically acceptable salt or metal complex. The pharmaceutical compositions of the invention may also contain other pharmaceutically active ingredients.

In the case of oral preparations, the pharmaceutical compositions of the invention may contain the dipeptide or salt or complex thereof alone in pure form or in admixture with an appropriate pharmaceutical additive such as a carrier or diluent, for formulation into a tablet, powder, granulate or capsule, e.g. with one or more conventional additives such as lactose, mannitol, corn starch or potato starch; with a binder such as crystalline cellulose, a cellulose derivative, acacia, corn starch or gelatin; with a disintegrator such as corn starch, potato starch or sodium carboxymethylcellulose; with a lubricant such as talc or magnesium stearate; and, if desired, with a diluent, buffering agent, moistening agent, preservative and flavouring agent.

Furthermore, the dipeptide can be encapsulated into a liposome prepared from a suitable lipid, for example a phospholipid such as lecithin, sphingomyelin, phosphatidyl ethanolamine, phosphatydil serine, cholesterol, phosphatidic acid, dicetyl phosphate, stearylamine, etc. The liposome can possess either a multi-layered or mono-layered structure and can also encapsulate a stabilizer, buffering agent, etc. Furthermore, the liposome can be made up into capsule form.

For parenteral administration, the dipeptide of the invention can be formulated into an injectable preparation by dissolving, suspending or emulsifying in an aqueous or non-aqueous solvent, such as distilled water for injection, physiological saline, Ringer's solution, vegetable oil, synthetic aliphatic acid glycerides, the esters of higher aliphatic acids or propylene glycol, and can contain preser vatives, stabilizers, buffering agents or solubilizers as required. Furthermore, the dipeptide can be presented in the form of a dried powder for injection by dissolving it in a liquid immediately prior to use, or as a suppository, ointment, etc. by mixing with suitable bases.

The dose of dipeptide or salt or metal complex thereof in the pharmaceutical composition of the present invention varies with the subject, form of the composition, and method and period of administration. The recommended adult oral dosage is from 1 to 2000 mg, preferably from 20 to 1200 mg. For parenteral administration, e.g. injections, one tenth to one third of the oral dose is recommended daily.

The invention will now be further illustrated with reference to the following examples of phamaceutical preparations.

## Example 1   (injectable preparation)

| Component | Content per ampoule (mg) |
| --- | --- |
| Dipeptide of the invention | 10 |
| Distilled water for injection | as required |
| Sodium chloride | as required |
| Total | 1 ml |

Example 2 (tablet)

| Component | Content per tablet (mg) |
|---|---|
| Dipeptide of the invention | 50 |
| Lactose | 190 |
| Crystalline cellulose | 50 |
| Magnesium stearate | 10 |
| Total | 300 mg |

Example 3 (capsule)

| Component | Content per capsule (mg) |
|---|---|
| Dipeptide of the invention | 50 |
| Lactose | 250 |
| Total | 300 mg |

**Claims**

1. A pharmaceutical composition containing as an active ingredient a linear or cyclic dipeptide composed of two amino acid residues selected from glycine (Gly), serine (Ser), histidine (His) and lysine (Lys), or a pharmaceutically acceptable salt thereof, with the proviso that linear dipeptides having His at the amino terminus and Gly-Gly are excluded.

2. A pharmaceutical composition as claimed in claim 1, wherein the amino acid residue at the amino terminus is Gly or Ser and that at the carboxyl terminus is Ser, His or Lys.

3. A pharmaceutical composition as claimed in claim 2, wherein the dipeptide is a linear dipeptide.

4. A pharmaceutical composition as claimed in claim 3, wherein the dipeptide is Gly-Ser or Ser-His.

5. A pharmaceutical composition as claimed in claim 1, wherein the dipeptide is a cyclic dipeptide.

6. A pharmaceutical composition as claimed in claim 5, wherein the dipeptide is cyclic His-Lys.

7. A pharmaceutical composition as claimed in any one of the preceding claims for the treatment of ulcers.

8. The use of dipeptide composed of two amino acid residues selected from Gly, Ser, His and Lys, or a pharmaceutically acceptable salt thereof, with the proviso that linear dipeptides having His at the amino terminus and Gly-Gly are excluded, for the manufacture of a pharmaceutical composition.

9. The use of dipeptide composed of two amino acid residues selected from Gly, Ser, His and Lys, or a pharmaceutically acceptable salt thereof, with the proviso that linear dipeptides having His at the amino terminus and Gly-Gly are excluded, for the manufacture of an anti-ulcer agent.

**Revendications**

5

1. Composition pharmaceutique comprenant comme substance active un dipeptide linéaire ou cyclique composé de deux résidus d'amino-acides, sélectionnés parmi la glycine (Gly), la sérine (Ser), l'histidine (His) et la lysine (Lys), ou un sel pharmaceutiquement acceptable de les derniers, les dipeptides linéaires ayant de l'histidine à la terminaison amino et de la glycine-glycine (Gly-Gly) étant exclus.

2. Composition pharmaceutique selon la revendication 1, dans laquelle le résidu d'amino-acide à la terminaison amino est de la glycine ou de la sérine et celui à la terminaison carboxyle est de la sérine (Ser), de l'histidine (His) ou la lysine (Lys).

3. Composition pharmaceutique selon la revendication 2, dans laquelle le dipeptide est un dipeptide linéaire.

4. Composition pharmaceutique selon la revendication 3, dans laquelle le dipeptide est de la glycine-sérine (Gly-Ser) ou de la sérine-histidine (Ser-His).

5. Composition pharmaceutique selon la revendication 1, dans laquelle le dipeptide est un dipeptide cyclique.

6. Composition pharmaceutique selon la revendication 5, dans laquelle le dipeptide est de l'histidine-lysine (His-Lys) cyclique.

7. Composition pharmaceutique suivant l'une quelconque des revendications précédentes pour le traitement des ulcères.

8. Utilisation d'un dipeptide composé de deux résidus d'amino-acides, sélectionnés parmi la glycine (Gly), la sérine (Ser), l'histidine (His) et la lysine (Lys), ou un sel pharmaceutiquement acceptable de les derniers, les dipeptides linéaires ayant de l'histidine à la terminaison amino et de la glycine-glycine étant exclus, pour produire une composition pharmaceutique.

9. Utilisation d'un dipeptide composé de deux résidus d'amino-acides, sélectionnés parmi la glycine (Gly), la sérine (Ser), l'histidine (His) et la lysine (Lys), ou un sel pharmaceutiquement acceptable de les derniers, les dipeptides linéaires ayant de l'histidine à la terminaison amino et de la glycine-glycine (Gly-Gly) étant exclus, pour produire un agent anti-ulcère.

**Patentansprüche**

1. Arzneimittelzubereitung, die als Wirkstoff ein lineares oder zyklisches Dipeptid enthält, zusammengesetzt aus zwei Aminosäureresten, ausgewählt aus Glyzin (Gly), Serin (Ser), Histidin (His) und Lysin (Lys), oder ein pharmazeutisch annehmbares Salz davon, unter der Bedingung, dass lineare Dipeptide mit N-terminalem Histidin und Gly-Gly ausgeschlossen sind.

2. Arzneimittelzubereitung nach Anspruch 1, worin die N-terminale Aminosäure Gly oder Ser ist und die C-terminale Ser, His oder Lys.

3. Arzneimittelzubereitung nach Anspruch 2, worin das Dipeptid ein lineares Dipeptid ist.

4. Arzneimittelzubereitung nach Anspruch 3, worin das Dipeptid Gly-Ser oder Ser-His ist.

5. Arzneimittelzubereitung nach Anspruch 1, worin das Dipeptid ein zyklisches Dipeptid ist.

6. Arzneimittelzubereitung nach Anspruch 5, worin das Dipeptid ein zyklisches His-Lys ist.

7. Arzneimittelzubereitung nach einem oder mehreren der vorangegangenen Ansprüche zur Behandlung von Geschwüren.

8. Verwendung von Dipeptiden, zusammengesetzt aus zwei Aminosäureresten, ausgewählt aus Gly, Ser, His und Lys, oder ein pharmazeutisch annehmbares Salz davon, unter der Bedingung, dass lineare Dipeptide mit N-terminalem His und Gly-Gly ausgeschlossen sind, zur Herstellung einer Arzneimittelzubereitung.

9. Verwendung von Dipeptiden, zusammengesetzt aus zwei Aminosäureresten, ausgewählt aus Gly, Ser, His und Lys, oder ein pharmazeutisch annehmbares Salz davon, unter der Bedingung, dass lineare Dipeptide mit N-terminalem His und Gly-Gly ausgeschlossen sind, zur Herstellung eines Mittels gegen Geschwüre.